# EUROPEAN PATENT APPLICATION

(11) **EP 4 552 636 A1**
(43) Date of publication of application: **14.05.2025**
(21) Application number: 23870949.7
(22) Date of filing: 27.09.2023
(51) Int. Cl.: A61K 31/165, A61K 9/70, A61P 19/06

(54) **PATCH COMPRISING COLCHICINE AND PREPARATION METHOD THEREFOR AND USE THEREOF**

(30) Priority: 30.09.2022 CN 202211213655
(71) Applicant: DEMOTECH.INC., Beijing 100176 (CN)
(72) Inventor: XIE, Wenwei, Beijing 100176 (CN); LU, Song, Beijing 100176 (CN); SUN, Lina, Beijing 100176 (CN); WANG, Yuan, Beijing 100176 (CN)
(74) Representative: ZBM Patents - Zea, Barlocci & Markvardsen
(86) International application number: PCT/CN2023/122148
(87) International publication number: WO 2024/067722

(57) **Abstract**

Provided are a patch containing colchicine, a preparation method and a use thereof, where the patch includes a polymer matrix layer. The polymer matrix layer includes the following components in weight percentage: 0.1%-5.0% of colchicine, 0.005%-15% of a permeation enhancer, and 80%-99% of a pressure-sensitive adhesive. The patch containing colchicine can achieve transdermal permeation of an amount sufficient for achieving treatment effect, and features no local irritant during pasting and after pasting, no gastrointestinal side effects, and high patient compliance.

## Description

### CROSS-REFERENCE TO RELATED APPLICATION

The present disclosure claims priority of Chinese Patent Application No. 202211213655.8, filed on September 30, 2022, entitled "PATCH CONTAINING COLCHICINE AND PREPARATION METHOD AND USE THEREOF", the entire disclosure content of which is incorporated herein by reference in its entirety.

### TECHNICAL FIELD

The present disclosure belongs to the field of percutaneous drug delivery technology, and specifically relates to a patch containing colchicine, a preparation method and a use thereof.

### BACKGROUND

Colchicine, which has a chemical name of N-(5,6,7,9-tetrahydro-1,2,3,10-tetramethoxy-9-oxobenzo[α]cycloheptatrienocycloheptatrien-7-yl)-(S)-acetamide, has a molecular formula of C₂₂H₂₅NO₆ and a molecular weight of 399.4. It is a light yellow to light greenish-yellow amorphous or crystalline powder, odorless, and darkens upon exposure to light. Its chemical structure is as follows:

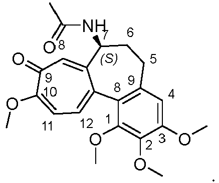

Colchicine has a special tricyclic structure, which is unstable under high temperature and light, especially, it produces genotoxic impurities C and G under light. The chemical structures of the impurities C and G are as follows:

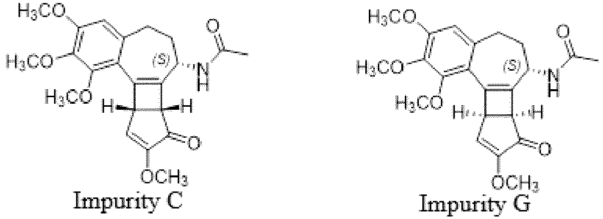

Colchicine, as a natural plant alkaloid, has been used by human beings since BC, and pure products were extracted from colchicum plants for the first time in 1820. Colchicine is currently approved by major drug regulatory agencies such as FDA, PDMA, EMA and NPMA, such as for the prevention and treatment of gout. Gout has become the second largest metabolic disease after diabetes, and colchicine is currently the first-line treatment and prevention drug during acute attacks of gout. Besides gout, colchicine is also used in the treatment of other diseases, such as Mediterranean fever, treatment and prevention of cardiovascular diseases such as pericarditis, skin diseases such as Sweet's syndrome and psoriasis.

However, colchicine has a small treatment safety window because its therapeutic dose is close to its toxic dose; moreover, it exhibits low bioavailability due to the first-pass effect in the liver. The currently approved oral colchicines all have serious side effects, such as abdominal pain, nausea, vomiting, diarrhea, bone marrow suppression, arrhythmia and azoospermia. Therefore, although colchicine has high clinical effectiveness, these serious side effects prevent more than 80% patients from insisting on oral colchicine treatment.

Transdermal drug delivery system (TDDS) mainly refers to a mode of drug delivery in which drugs play a pharmaceutical role either locally at the site of treatment or systemically throughout the body after being absorbed through skin or mucosa. Transdermal drug delivery has many advantages, such as no gastrointestinal irritation, avoiding the first-pass effect in the liver, non-invasiveness, long-term sustained release, reducing the frequency of frequent administration and avoiding the fluctuation in blood drug concentration caused by oral absorption, thus having higher safety and good patient compliance.

But so far, although some attempts to deliver colchicine through transdermal drug delivery have been reported in the literature, the demand for higher safety and better compliance of colchicine has not been solved clinically, so there is an urgent need for a transdermal drug delivery system that can deliver colchicine safely and effectively.

### SUMMARY

The technical problem to be solved by the present disclosure is to overcome the shortcomings in the prior art and provide a patch containing colchicine, a preparation method and a use thereof, where the patch includes a polymer matrix layer. The polymer matrix layer includes the following components in weight percentage: 0.1%-5.0% of colchicine, 0.005%-15% of a permeation enhancer, and 80%-99% of a pressure-sensitive adhesive. The patch containing colchicine in the present disclosure can achieve transdermal permeation of an amount sufficient for achieving treatment effect, and features no local irritant during pasting and after pasting, no gastrointestinal side effects, and high patient compliance.

In order to solve the above technical problems, the following technical solutions are used in the present disclosure:

In one aspect, the present disclosure provides a patch containing colchicine, where the patch includes a polymer matrix layer. The polymer matrix layer includes the following components in weight percentage:

| | |
|---|---|
| colchicine | 0.1%-5.0%, |
| a permeation enhancer | 0.005%-15%, and |
| a pressure-sensitive adhesive | 80%-99%. |

Preferably, in the polymer matrix layer, a weight percentage of the colchicine is 0.25%-4.0%, further preferably 0.50%-3.5%, and more preferably 0.50%-3.0%, which more specifically can be 0.50%, 1.0%, 1.5%, 2.0%, 2.5%, and 3.0%.

Preferably, in the polymer matrix layer, a weight percentage of the permeation enhancer is 0.01%-12%, which more specifically can be 0.05%, 0.10%, 0.15%, 0.20%, 0.25%, 0.50%, 0.70%, 1.00%, 1.50%, 2.00%, 3.00%, 5.00%, 6.00%, 7.00%, 8.00%, 9.00%, and 10.00%.

Preferably, in the polymer matrix layer, a weight percentage of the pressure-sensitive adhesive is 85%-98%, which more specifically can be 85%, 90%, 95%, and 98%.

Preferably, the patch containing colchicine further includes a backing layer; and
preferably, the patch containing colchicine further includes a protective layer, and the polymer matrix layer is located between the backing layer and the protective layer.

Preferably, the permeation enhancer is selected from reagents capable of dissolving colchicine and having a boiling point greater than or equal to 150°C;
preferably, the permeation enhancer is selected from one or more of alcohol, alcohol amine and alcohol ether;
preferably, the permeation enhancer has a boiling point greater than or equal to 180°C;
preferably, the alcohol is selected from monohydric alcohol and/or dihydric alcohol;
preferably, the monohydric alcohol is selected from one or more of lauryl alcohol, oleyl alcohol and terpineol;
preferably, the dihydric alcohol is selected from hexanediol;
preferably, the alcohol amine is selected from one or more of tromethamine, ethanolamine, diethanolamine, triethanolamine, N-hydroxyethyl piperidine, N-hydroxyethyl pyrrolidine, N-hydroxyethyl piperazine, N,N-dibutylaminoethanol and N,N-diethylaminoethanol; and
preferably, the alcohol ether is selected from one or more of diethylene glycol monoethyl ether, diethylene glycol monobutyl ether, diethylene glycol monohexyl ether, dipropylene glycol methyl ether, dipropylene glycol butyl ether and dipropylene glycol.

Preferably, the backing layer is selected from a closed backing film or a non-closed backing film;
preferably, the backing layer is selected from the closed backing film;
preferably, the closed backing film is selected from a light-proof closed backing film;
preferably, the closed backing film is selected from a polyester-polyethylene composite film lined with metal aluminum;
preferably, the closed backing film has a thickness of 20-100 µm; and
preferably, the protective layer is selected from a release film.

Preferably, the pressure-sensitive adhesive is selected from one or more of acrylic pressure-sensitive adhesives or silicone pressure-sensitive adhesives; and more preferably, the pressure-sensitive adhesive is selected from one or two of acrylic pressure-sensitive adhesive or silicone pressure-sensitive adhesive.

Preferably, the polymer matrix layer includes colchicine with a content ranging from 10 µg/cm² to 150 µg/cm²; and
preferably, an administration area of the patch containing colchicine is 5 cm²-100 cm².

In another aspect, the present disclosure provides a method for preparing a patch containing colchicine as described in the present disclosure, including the following Steps:
(1) dissolving a prescribed amount of colchicine and a permeation enhancer in an organic solvent to obtain a mixed solution;
(2) dropping the mixed solution obtained in Step (1) into a prescribed amount of a pressure-sensitive adhesive to obtain an adhesive solution;
(3) coating the adhesive solution obtained in Step (2) on a protective layer, and removing the organic solvent; and
(4) compositing a product obtained in Step (3) with a backing layer, and die-cutting it to obtain the patch containing colchicine.

In yet another aspect, the present disclosure provides a method for preparing a patch containing colchicine as described in the present disclosure, including the following Steps:
(1') adding and mixing a prescribed amount of colchicine, a permeation enhancer and a pressure-sensitive adhesive into an organic solvent to obtain an adhesive solution;
(2') coating the adhesive solution obtained in Step (1') on a protective layer, and removing the organic solvent; and
(3') compositing a product obtained in Step (2') with a backing layer, and die-cutting it to obtain the patch containing colchicine.

Preferably, the organic solvent in the Step (1) and/or the Step (1') is selected from one or more of methanol, ethanol or isopropanol; and
preferably, a specific operation of removing the organic solvent in the Step (3) and/or (2') is: removing the organic solvent by drying at 35-50°C.

In further another aspect, the present disclosure provides a use of a patch containing colchicine as described in the present disclosure in preparing a medicament for preventing and/or treating gout and/or pericarditis.

The present disclosure further provides a method for treating gout and/or pericarditis, including administrating a therapeutically effective amount of a patch containing colchicine as described in the present disclosure to a subject in need thereof.

The present disclosure further provides a patch containing colchicine, for use in preventing and/or treating gout and/or pericarditis.

By usage of the technical solutions above, compared with the prior art, the present disclosure has the following advantages:
1. The colchicine content in the patch containing colchicine in the present disclosure features no skin local irritant during pasting and after pasting, no gastrointestinal side effects, and high patient compliance while achieving transdermal permeation of an amount sufficient for achieving treatment effect;
2. The permeation enhancer of the present disclosure can promote colchicine to transdermal permeation of an amount sufficient for achieving treatment effect continuously and controllably, and has good compatibility with the colloid matrix;
3. The patch containing colchicine of the present disclosure includes a backing layer directly connected with the polymer matrix layer, and the backing layer plays a role in protecting the polymer matrix layer from contacting with the surrounding environment and preventing drug loss when in use;
4. The backing layer of the present disclosure is preferably a closed light-proof backing film. The closed backing film has a good controllable permeation performance, and the colloidal matrix remains in a good state after pasting, thus making the closed backing film maintain a good pasting performance, and meet the wearing requirements of the patch during use. The light-proof backing film can reduce or eliminate and prevent the risk of colchicine photolysis.

### BRIEF DESCRIPTION OF THE DRAWINGS

FIG. 1a is a state diagram of colchicine with different contents dissolved in the 4098 pressure-sensitive adhesive for 0 days;
FIG. 1b is a state diagram of colchicine with different contents after being placed in the 4098 pressure-sensitive adhesive at room temperature for 4 months;
FIG. 2 is a diagram of the results of *in vitro* permeation test of the patch of Example 24;
FIG. 3 is a diagram of the results of *in vivo* permeation test of the patch of Example 24;
FIG. 4 shows the concentrations of various tissues or plasma in male Bama miniature pigs after a single transdermal administration of the patch containing colchicine of the present disclosure at a dose of 11.9 mg/ pig;
FIG. 5 and FIG. 6 are partial enlarged views of FIG. 4, respectively; and
FIG. 7 shows the concentrations of various tissues or plasma in male Bama miniature pigs after a single gavage administration of the colchicine tablets at a dose of 1 mg/ pig.

### DESCRIPTION OF THE EMBODIMENTS

The present disclosure specifically provides a patch containing colchicine, including a polymer matrix layer. The polymer matrix layer includes the following components in weight percentage:

| | |
|---|---|
| colchicine | 0.1%-5.0%, |
| a permeation enhancer | 0.005%-15%, and |
| a pressure-sensitive adhesive | 80%-99%. |

Colchicine has certain skin irritant, which is not only related to the composition of the colloidal matrix, including but not limited to the types and contents of the permeation enhancer, and the types and contents of the pressure-sensitive adhesive, but also directly related to the content of colchicine. Therefore, in order to reduce or eliminate the local skin irritant of the patch containing colchicine at the pasting site while ensuring the permeation of the therapeutic amount, in some specific embodiments, the ratio of the weight of colchicine to the dry weight of the polymer matrix layer is 0.1%-5.0%, preferably 0.25%-4.0%, further preferably 0.50%-3.5%, more preferably 0.50%-3.0%, and more specifically can be 0.50%, 1.0%, 1.5%, 2.0%, 2.5%, and 3.0%. With the above content, the patch containing colchicine can ensure continuous and controlled delivery of an amount sufficient for achieving treatment effect during pasting, while reducing or eliminating the production of the local skin irritant.

In order to penetrate an amount sufficient for achieving treatment effect, the present disclosure includes a permeation enhancer. Not all types of permeation enhancers can achieve the purpose of the present disclosure, because colchicine has excellent water solubility due to its special structure, which is generally soluble in alcohols and their derivatives, but insoluble in solvents with lower polarity such as ethyl acetate. The pressure-sensitive adhesive system used in the present disclosure employs ethyl acetate as the main system. Therefore, a problem to be solved by the present disclosure is to select a suitable permeation enhancer which can promote the transdermal permeation of a dose of colchicine sufficient for achieving treatment effect, while uniformly dissolving or dispersing colchicine in a colloidal matrix with ethyl acetate as the main solvent system.

In some specific embodiments, the permeation enhancer includes but is not limited to alcohol such as methanol, ethanol, diethanol, terpineol, oleyl alcohol, camphor, borneol, etc., alcohol ether such as diethylene glycol monomethyl ether, etc., alcohol amine such as triethanolamine, N-hydroxyethyl pyrrolidine, etc., and others such as dimethyl sulfoxide (DMSO), N-methylpyrrolidone and oleic acid, etc.

The present disclosure unexpectedly found that in a transdermal drug delivery system, while some permeation enhancers have a permeation enhancing effect, they also pose a potential risk of destroying the colloidal performance of pressure-sensitive adhesives, especially in the case of adding higher content, for example, in some special embodiments, after adding some permeation enhancers, the pressure-sensitive adhesive in solution is partially transformed into gel state or partially lumpy, thus changing the colloidal rheology and further leading to coating failure; and in some special embodiments, as the content of the permeation enhancer increases, the colloidal performance will be further destroyed. Therefore, the present disclosure selects a permeation enhancer based on the consideration of both the permeation enhancing effect and the colloid performance. The present disclosure finds that some types of permeation enhancers have good compatibility with the colloidal matrix and can play the expected effect of increasing diffusion speed.

In some specific embodiments, the permeation enhancer is selected from reagents capable of dissolving colchicine and having a boiling point greater than or equal to 150°C.

In some specific embodiments, the permeation enhancer is selected from one or more of alcohol, alcohol amine and alcohol ether.

In some specific embodiments, the permeation enhancer has a boiling point greater than or equal to 180°C.

In some specific embodiments, the alcohol is selected from monohydric alcohol and/or dihydric alcohol.

In some specific embodiments, the monohydric alcohol includes but is not limited to lauryl alcohol, oleyl alcohol, terpineol, etc., the dihydric alcohol includes but is not limited to hexanediol, etc., the alcohol amine includes but is not limited to tromethamine, ethanolamine, diethanolamine, triethanolamine, N-hydroxyethyl piperidine, N-hydroxyethyl pyrrolidine, N-hydroxyethyl piperazine, N,N-dibutylaminoethanol, N,N-diethylaminoethanol, etc., and the alcohol ether includes but is not limited to diethylene glycol monoethyl ether, diethylene glycol monobutyl ether, diethylene glycol monohexyl ether, dipropylene glycol methyl ether, dipropylene glycol butyl ether, dipropylene glycol, etc.

In some specific embodiments, the permeation enhancer can be used alone or in combination.

The permeation enhancer not only has a permeation enhancing effect on colchicine, but also the monohydric alcohol, dihydric alcohol, alcohol amine and alcohol ether permeation enhancer has the ability to increase the solubility of colchicine in the colloidal matrix, so the solubility of colchicine in the colloidal matrix tends to increase after adding permeation enhancers. Another aspect of the increase in solubility means that colchicine with the same content is farther away from reaching or approaching its maximum saturated solubility. However, the thermodynamic passive diffusion ability of colchicine is directly related to the degree of approaching saturated solubility. Generally, it has the highest thermodynamic passive diffusion ability when it reaches or approaches the maximum saturated solubility. Therefore, the addition of a solubilizing permeation enhancer increases the solubility of colchicine in the colloidal matrix, thereby reducing its passive thermodynamic diffusion ability to a certain extent.

Therefore, the permeation enhancing ability is not only related to the type of a permeation enhancer, but also to its content. Therefore, in certain special embodiments, on the contrary, the addition of a permeation enhancer reduces the permeation ability and does not play a role in permeation enhancing. Although the permeation ability can be increased by the commonly known method in the field, i.e., further increasing the content of colchicine so as to reach or approach the saturated solubility again, reaching or approaching the saturated solubility so as to increase the permeation ability by further increasing the content range of colchicine in the colloidal matrix is limited because of the skin local irritant related to the content of colchicine.

In some specific embodiments, the ratio of the weight of the permeation enhancer to the dry weight of the polymer matrix layer is 0.005%-15%, preferably 0.01%-12%, and specifically can be 0.05%, 0.10%, 0.15%, 0.20%, 0.25%, 0.50%, 0.70%, 1.00%, 1.50%, 2.00%, 3.00%, 5.00%, 6.00%, 7.00%, 8.00%, 9.00%, and 10.00%.

The pressure-sensitive adhesive is selected from a pressure-sensitive adhesive suitable for a transdermal drug delivery system pharmaceutically. In some specific embodiments, it is selected from one of acrylic pressure-sensitive adhesive based on acrylic polymer compound or silicone pressure-sensitive adhesive based on silicone polymer compound.

In some specific embodiments, the acrylic pressure-sensitive adhesive can be any homopolymer, copolymer, trimer and polymer of different acrylic acids. According to the functional groups included, the acrylic pressure-sensitive adhesive can be non-functionalized acrylic pressure-sensitive adhesive, and functionalized acrylic pressure-sensitive adhesive, including but not limited to including functional groups such as hydroxyl group, carboxyl group, or both hydroxyl group and carboxyl group, amino group and epoxy group. **In** some embodiments, based on the amount of the active ingredient of colchicine added and the amount to be delivered sufficient for achieving treatment effect, the types and dosages of the acrylic pressure-sensitive adhesives are different, and functionalized acrylic pressure-sensitive adhesive, non-functionalized acrylic pressure-sensitive adhesive, or a mixture of the two can be selected.

In some specific embodiments, the ratio of the weight of the acrylic pressure-sensitive adhesive to the dry weight of the polymer matrix layer is 80%-99%, preferably 85%-98%, and specifically can be 85%, 90%, 95%, and 98%.

Commercially available acrylic pressure-sensitive adhesives comprise Duro-Tak products from Henkel, such as Duro-Tak 87-900A, Duro-Tak 87-9301 (a non-crosslinked vinyl acetate-free acrylic pressure-sensitive adhesive without functional groups), Duro-Tak 87-4098 (a non-crosslinked vinyl acetate acrylic pressure-sensitive adhesive without functional groups), Duro-Tak 87-2287 (a non-crosslinked vinyl acetate acrylic pressure-sensitive adhesive with a hydroxyl functional group), Duro-Tak 87-2852 (a crosslinked acrylic pressure-sensitive adhesive with a carboxyl functional group), Duro-Tak 87-2196 (a crosslinked acrylic pressure-sensitive adhesive with a carboxyl functional group), Duro-Tak 87-2296 (a crosslinked acrylic pressure-sensitive adhesive with a carboxyl functional group), Duro-Tak 87-2194 (a crosslinked acrylic pressure-sensitive adhesive with a carboxyl functional group), Duro-Tak 87-2516 (a crosslinked acrylic pressure-sensitive adhesive with a carboxyl functional group), Duro-Tak 87-2070 (a crosslinked acrylic pressure-sensitive adhesive with a carboxyl functional group), Duro-Tak 87-2353 (a non-crosslinked acrylic pressure-sensitive adhesive with a carboxyl functional group), Duro-Tak 87-2154 (a crosslinked acrylic pressure-sensitive adhesive with a carboxyl functional group) and Duro-Tak 87-2510 (a non-crosslinked acrylic pressure-sensitive adhesive with a hydroxyl functional group).

In some specific embodiments, the ratio of the weight of the silicone pressure-sensitive adhesive to the dry weight of the polymer matrix layer is 90%-99%, preferably 95%-99%, and specifically can be 95%, 96%, 97%, 98%, and 99%.

Commercially available silicone pressure-sensitive adhesives comprise products from DuPont, such as Liveo^{™} 7-6102 SilAc Hybrid PSA, Liveo^{™} 7-6302 SilAc Hybrid PSA, Liveo^{™} BIO-PSA 7-4502, Liveo^{™} BIO-PSA 7-4602, Liveo^{™} BIO-PSA7-4302 and Liveo^{™} BIO-PSA 7-4102.

The physical and chemical properties of colchicine result in its higher solubility in an acrylic pressure-sensitive adhesive, and lower solubility in a silicone pressure-sensitive adhesive. In some specific embodiments, the pressure-sensitive adhesive can be selected from the mixture of acrylic pressure-sensitive adhesive and silicone pressure-sensitive adhesive. When using the mixture of acrylic pressure-sensitive adhesive and silicone pressure-sensitive adhesive, the comprehensive consideration of the permeation ability of colchicine and safety can be achieved by adjusting the mixing ratio of them. The mixing method of acrylic pressure-sensitive adhesive and silicone pressure-sensitive adhesive can be through physical mixing, that is, two kinds of pressure-sensitive adhesives with different properties are mixed and stirred into a uniformly dispersed suspension; the mixed acrylic and silicone pressure-sensitive adhesive can also be obtained by a chemical synthesis method.

In some specific embodiments, the ratio of the weight of the pressure-sensitive adhesive composed of the acrylic pressure-sensitive adhesive and the silicone pressure-sensitive adhesive to the dry weight of the polymer matrix layer is 90%-99%, preferably 95%-99%, and specifically can be 95%, 96%, 97%, 98%, and 99%. The dry adhesive weight ratio of acrylic pressure-sensitive adhesives to silicone pressure-sensitive adhesives can be 30-70:70-30, and specifically can be 50:50, 40:60, 60:40, 30:70 or 70:30.

In some specific embodiments, the patch containing colchicine further includes a backing layer and a protective layer, and the polymer matrix layer is located between the backing layer and the protective layer.

The backing layer is directly connected with one surface of the polymer matrix layer, and the backing layer plays a role in protecting the polymer matrix layer from contacting with the surrounding environment and preventing drug loss when in use.

The protective layer is selected from a release film and directly connected with the other surface of the polymer matrix layer. The release film is removed before using the patch.

In some specific embodiments, the backing layer is selected from a pharmaceutically acceptable backing film, including but not limited to a polyester, polyester and vinyl acetate composite film, a polyethylene and ethyl acetate composite film, polyurethane, polyester-polyethylene composite film lined with metal foil such as metal aluminum, a non-woven fabric, an elastic fabric, etc.

Due to the good water solubility of colchicine, water from skin's respiration at the pasting site during pasting enters the colloidal matrix, thereby affecting the solubility of such watersoluble components in the colloidal matrix and subsequently influencing its thermodynamic diffusion ability, i.e., permeation ability, and pasting performance. The extent of this influence depends on the pasting duration and the nature of the backing film.

Therefore, in order to solve the influence of the moisture generated by skin's respiration during pasting on the permeation behavior and pasting performance of the patch. **In** some specific embodiments, the backing layer is selected from a closed backing film or a non-closed backing film.

A closed backing film and a non-closed backing film are classified according to their permeation abilities to water and oxygen. The non-closed backing film has certain permeation ability to oxygen and water from skin's respiration, such as non-woven fabrics and elastic fabrics, which have the relatively highest permeation to water and oxygen; the closed backing film has a blocking effect on oxygen and water from skin's respiration, such as a polyester-polyethylene composite film lined with metal aluminum, which has extremely low water and oxygen permeation abilities. According to the property and thickness of the backing film material, the permeation rate of water and oxygen can be adjusted.

In some specific embodiments, the backing layer is selected from a non-closed backing film. The non-closed backing film facilitates the discharge of water and reduces the decrease of the thermodynamic permeation ability of colchicine, thus maintaining the permeation ability. However, after a non-closed backing film is pasted for a certain period of time, the colloidal matrix will partially remain on the skin, resulting in peeling failure.

In some specific embodiments, the backing layer is selected from a closed backing film. The closed backing film has a good controllable permeation performance, and the colloidal matrix remains in a good state after pasting, thus maintaining a good pasting performance. The closed backing film has a thickness of 20-100 µm, preferably 20-80 µm.

In some specific embodiments, the closed backing film is selected from a polyester-polyethylene composite film lined with metal aluminum, which has a thickness of 20-100 µm, preferably 20-80 µm.

In some specific embodiments, the backing film is selected from ScotchPak^{™}1109, ScotchPak^{™}9738 or ScotchPak^{™}9730 from 3M, and preferably ScotchPak^{™}9738.

In some specific embodiments, the polymer matrix layer includes colchicine with a content ranging from 10 µg/cm² to 150 µg/cm². There can also be other ranges, specifically 15 µg/cm²-120 µg/cm², 20 µg/cm²-100 µg/cm², 20 µg/cm²-25 µg/cm², 30 µg/cm²-35 µg/cm², and more specifically, the ranges can be 20 µg/cm², 25 µg/cm², 30 µg/cm², 35 µg/cm², 40 µg/cm², 45 µg/cm², 50 µg/cm², 55 µg/cm², 60 µg/cm², 65 µg/cm², 70 µg/cm², 75 µg/cm², 80 µg/cm², 85 µg/cm², 90 µg/cm², and 100 µg/cm².

In some specific embodiments, according to the blood drug concentration or clinical effect to be achieved within the pasting time range, an administration area of the patch containing colchicine is 5 cm²-100 cm². There can also be other ranges, specifically, 10 cm²-90 cm², and more specifically, the ranges can be 10 cm², 15 cm², 20 cm², 30 cm², 40 cm², 50 cm², 60 cm², 70 cm², and 80 cm².

In some specific embodiments, the present disclosure provides a method for preparing a patch containing colchicine as described in the present disclosure, including the following Steps:
(1) dissolving a prescribed amount of colchicine and a permeation enhancer in an organic solvent to obtain a mixed solution, where the weight ratio of colchicine to the organic solvent is 1:1;
(2) uniformly stirring the mixed solution obtained in Step (1), and then dropping into a prescribed amount of a pressure-sensitive adhesive to obtain an adhesive solution;
(3) uniformly stirring the adhesive solution obtained in Step (2), and then coating the stirred adhesive solution on a protective layer (a release film), drying the coated polymer matrix layer at a certain temperature to remove the organic solvent; and
(4) compositing a product obtained in Step (3) with a backing layer to obtain a composite product, and die-cutting the composite product into required specifications according to a requirement to obtain the patch containing colchicine.

In some specific embodiments, the present disclosure provides a method for preparing a patch containing colchicine as described in the present disclosure, including the following Steps:
(1') adding and mixing a prescribed amount of colchicine, a permeation enhancer and a pressure-sensitive adhesive into an organic solvent to obtain an adhesive solution;
(2') uniformly stirring the adhesive solution obtained in Step (1'), and then coating the stirred adhesive solution on a protective layer (a release film), drying the coated polymer matrix layer at a certain temperature to remove the organic solvent; and
(3') compositing a product obtained in Step (2') with a backing layer to obtain a composite product, and die-cutting the composite product into required specifications according to a requirement to obtain the patch containing colchicine.

In some specific embodiments, the organic solvent in the Step (1) and/or the Step (1') is selected from one or more of methanol, ethanol or isopropanol, and preferably ethanol.

In some specific embodiments, a specific operation of removing the organic solvent in the Step (3) and/or the Step (2') is: drying the coated polymer matrix layer at 35-50°C to remove the organic solvent.

It should be noted that the sequence involved in the preparation of the patch, and parameters such as the addition amount of each component, the stirring time and rate described above vary according to the required end use purpose. The parameters can be adjusted as required.

An exemplary preparation method is as follows:
Step 1): firstly, weighing a prescribed amount of colchicine and a permeation enhancer, stirring them to dissolve in an appropriate amount of ethanol , and stirring for 15-60 minutes according to the addition amount to obtain a mixed solution;
Step 2): dropping the mixed solution obtained in Step 1) into a prescribed amount of a pressure-sensitive adhesive while keeping stirring, and continuing to stir for 10-60 minutes to obtain an adhesive solution;
Step 3): uniformly stirring the adhesive solution obtained in Step 2), and then coating the stirred adhesive solution on a release film with a coating thickness depending on the final clinical use, and removing the organic solvent by drying at 35-50°C for 5-15 minutes through an oven with an air exhaust function, with the specific drying temperature and time depending on the coating speed and solvent residue; and
Step 4): compositing a product obtained in Step 3) with a backing film to obtain a composite product, and die-cutting the composite product into required specifications according to a use requirement to obtain the patch containing colchicine.

Based on the general preparation method of the patch, other methods reported in the literature can also be used to prepare the patch of the present disclosure.

In some specific embodiments, a use of a patch of the present disclosure in preparing a medicament for preventing and/or treating gout and/or pericarditis is disclosed.

### Description of terms:

Unless otherwise defined, all technical and scientific terms used in the present disclosure have the same meanings as commonly used in the field to which the present disclosure belongs. For the purpose of illustrating this specification, the following definitions will be applied, and where appropriate, the terms used in a singular form will also include the plural form, and vice versa.

As used in the present disclosure, the term "percutaneous drug delivery" or "transdermal drug delivery" refers to a drug delivery mode in which an active ingredient is delivered into a local or systemic system through skin or mucosa. They have the same meaning in the patent of the present disclosure and can be used interchangeably.

As used in the present disclosure, the term "polymer matrix layer" or "colloidal matrix layer" refers to the material combination of the polymer pressure-sensitive adhesive, colchicine and any other pharmaceutically acceptable adjuvants included in the transdermal drug delivery system. Generally, the polymer matrix layer is located between the release film and the backing film. The polymer matrix layer is used as the drug delivery layer of the transdermal drug delivery system to form an adhesive-drug mixed transdermal drug delivery system. The polymer matrix layer or the colloidal matrix layer of the present disclosure has the same meaning and can be used interchangeably.

As used in the present disclosure, the term "percutaneous patch" or "transdermal drug delivery system" refers to a system for transdermal drug delivery including active ingredients, which generally includes a backing layer, a release film and a polymer matrix drug delivery layer located between the two layers. According to the combination mode of active ingredients and other components in the polymer matrix drug delivery layer, it can be generally classified into reservoir type and adhesive-drug mixed type. A transdermal drug delivery system can also be referred to as a patch or percutaneous patch for short, and these names can be used interchangeably in the present disclosure. The polymer matrix drug delivery layer generally includes an active ingredient, a permeation enhancer and/or other pharmaceutically acceptable excipients suitable for percutaneous drug delivery patches, including but not limited to pressure-sensitive adhesives, fillers, crosslinking agents, antioxidants, ultraviolet absorbers, antibacterial agents, etc.

As used in the present disclosure, the term "permeation ability" refers to the passive diffusion of a drug through skin or mucosa, and the driving force is the concentration difference of the active ingredient on both sides of the skin. The cumulative permeation amount per unit time and unit area can be used as the evaluation indicator of the permeation ability of a patch, which is generally denoted as flux, with the unit of µg/cm²/hr.

As used in the present disclosure, the term "pressure-sensitive adhesive" refers to a class of viscoelastic polymer materials, which when in contact with the surface of most other materials, can adhere to the surface with only a light press and maintain long-lasting adhesion. The pressure-sensitive adhesive generally includes two types, one is a pressure-sensitive adhesive itself, and the other can obtain the function of a pressure-sensitive adhesive by adding a tackifier or a plasticizer. The pressure-sensitive adhesive has satisfactory physical properties at room temperature, such as good skin adhesion, maintaining adhesion for a certain period of time, the ability to be peeled off without damaging the skin, and the ability to control the degree of cold flow, thus meeting the requirements for pasting. Generally, it includes an acrylic pressure-sensitive adhesive, a silicone pressure-sensitive adhesive and a rubber pressure-sensitive adhesive, as well as a new mixed pressure-sensitive adhesive formed through physical mixing or chemical binding of the pressure-sensitive adhesives above, in order to regulate the property of the pressure-sensitive adhesive to meet a specific requirement.

As used in the present disclosure, the term "backing layer" refers to the layer in a percutaneous drug delivery patch that cannot be penetrated by the drug. One surface of the backing layer is directly connected with the polymer matrix layer, and the backing layer plays a role in protecting the polymer matrix layer from contacting with the surrounding environment and preventing drug loss when in use. The raw material corresponding to the backing layer is generally referred to as the backing film. The materials of the backing layer generally include polyester, polyethylene polyvinyl acetate composite films, polyvinyl chloride, polyurethane, metal foil composite films, non-woven fabrics, elastic fabrics, etc., and the thickness is generally 10~200 µm. For example, ScotchPak^{™} 9730, 9701, 9720, 9723 from 3M Company in the United States, the elastic fabric 6015A from Xiaoshan in China, the non-woven fabric EW2080 and EW2083 from Vilene in Japan, or PE3601 from Shanghai Yingke can be used. Backing films with different properties have different physical and chemical properties, such as the ductility, air permeation ability, oxygen permeation ability, light-proof ability, etc., so as to be classified into closed backing films and non-closed backing films. The backing layer or backing film of the present disclosure has the same meaning and can be used interchangeably.

As used in the present disclosure, the term "light-proof closed backing film" refers to a class of backing film with extremely low permeation of water, oxygen, light, etc., and generally includes metal foils with different thicknesses, such as aluminum foil.

As used in the present disclosure, the term "release film" can also be referred to as a protective layer, which is directly connected with the other surface of the polymer matrix layer. The release film is removed before using the percutaneous patch.

As used in the present disclosure, the term "permeation of an effective dose" or "permeation of a dose sufficient for achieving treatment effect" or "effectiveness" refers to that the active ingredient can be delivered through the skin to achieve the required amount for local or systemic action during the use of the percutaneous patch, so as to achieve specific pharmacological effects, such as curing, alleviating or controlling diseases or symptoms. The terms in the present disclosure can be used interchangeably.

As used in the present disclosure, the term "colchicine" refers to a tropolone alkaloid existing in the Liliaceae plant colchicum, which can inhibit cell mitosis, thus playing a corresponding pharmacological role.

As used in the present disclosure, the term "permeation enhancer" refers to a substance that can change the diffusion rate of the active ingredient into the skin, and is usually miscible with the active ingredient and uniformly dispersed in the polymer matrix layer.

As used in the present disclosure, the term "pasting performance" refers to all processes including the peel for peeling the patch from the release film, the peel for peeling the patch from the pasting site after pasting, the tack, the shear, the cold flow, etc. The "peel" refers to the force used to remove the backing layer including the polymer matrix layer from the release film or remove the patch from the skin after pasting, and it is required that the peel should not be too large, so as to avoid peeling failure or colloid remaining on the skin; the "tack" refers to the infiltration degree of the backing layer including the polymer matrix layer with the pasting site, which reduces the risk of detachment during pasting; the "shear" refers to the degree of displacement of the patch at the pasting site, which reflects the pasting strength during pasting; and the "cold flow" refers to the viscoelastic creep of the polymer matrix layer, which can lead to the formation of black circles in the process of wearing the patch, may cause the patch to stick to the protective layer and packaging container during storage, thus affecting the safety and effectiveness of patients. Generally, it is necessary to balance the properties of patches such as the peel, the tack, the shear and the cold flow.

As used in the present disclosure, the term "colloidal character" refers to the characters of the pressure-sensitive adhesive solution formed by adding all the raw materials and adjuvants to the pressure-sensitive adhesive during the formulation development process, such as the change of rheology, whether it is suitable for coating performance, etc.

As used in the present disclosure, the term "about" means a range of ±20% of the numerical value after it. In some implementations, the term "about" means a range of ±10% of the numerical value after it. In some implementations, the term "about" means a range of ±5% of the numerical value after it.

To make the technical solutions and beneficial effects of the present disclosure more apparent and easier to be understood, the detailed description is provided below by being in conjunction with the attached drawings and enumerating specific embodiments. It should be understood that these embodiments are only used to illustrate the present disclosure and are not intended to limit the scope of the present disclosure. Where the specific technology or conditions are not specified in the embodiments, it is usually carried out according to the conventional technology or conditions described in the literature in the art or according to the product specification. Unless otherwise specified, the reagents, materials or instruments used are all conventional products that can be purchased through formal commercial channels.

In the following Examples and Test Examples, the models and abbreviations of pressure-sensitive adhesives are as shown in Table 1.

**Table 1 List of models and abbreviations of pressure-sensitive adhesives**

| No. | Full designation of pressure-sensitive adhesive model | Abbreviation | Supplier |
|---|---|---|---|
| 1 | DURO-TAK 87-4098 | 4098 | Henkel |
| 2 | DURO-TAK^{™} 387-2287 / 87-2287 | 2287 | Henkel |
| 3 | DURO-TAK 387-2510 / 87-2510 | 2510 | Henkel |
| 4 | DURO-TAK 387-2051 / 87-2051 | 2051 | Henkel |
| 5 | DURO-TAK 87-2852 | 2852 | Henkel |
| 6 | DURO-TAK 87-235A | 235A | Henkel |
| 7 | DURO-TAK 387-2516 / 87-2516 | 2516 | Henkel |
| 8 | DURO-TAK 87-2677 | 2677 | Henkel |
| 9 | DURO-TAK 87-2074 | 2074 | Henkel |
| 10 | DURO-TAK 87-2196 | 2196 | Henkel |
| 11 | DURO-TAK 87-900A | 900A | Henkel |
| 12 | Liveo^{™} BIO-PSA 7-4302 | 4302 | DuPont |
| 13 | Liveo^{™} 7-6302 SilIca Hybrid PSA | 6302 | DuPont |

Where No. 1-11 are all polyacrylic pressure-sensitive adhesives from Henkel. Liveo^{™} BIO-PSA 7-4302 is a silicone pressure-sensitive adhesive, and Liveo^{™} 7-6302 SilAc Hybrid PSA is a composite pressure-sensitive adhesive of polyacrylic acid and silicone, which is commercially available from DuPont. For the convenience of writing, the letter parts of each pressure-sensitive adhesive are omitted in the Examples and Test Examples of the present disclosure, and only abbreviated numbers are used instead.

In the following Examples and Test Examples, English abbreviations are as shown in Table 2.

**Table 2 List of English abbreviations**

| No. | Abbreviation | Chinese name |
|---|---|---|
| 1 | API | Colchicine |
| 2 | IPP | Isopropyl palmitate |
| 3 | PEG600 | Polyethylene glycol 600 |
| 4 | DGME | Diethylene glycol monoethyl ether |
| 5 | HEP | N-hydroxyethyl pyrrolidine |
| 6 | TEOA | Triethanolamine |
| 7 | DPG | Dipropylene glycol |
| 8 | DMI | Isosorbide dimethyl ether |

The patch containing colchicine of the present disclosure includes a backing layer, a protective layer and a polymer matrix layer, where the polymer matrix layer includes: colchicine, a permeation enhancer and a pressure-sensitive adhesive, and the polymer matrix layer is located between the backing layer and the protective layer. Where, the backing layer is selected from light-proof closed backing films, the protective layer is selected from release films, and the permeation enhancer plays a role in dissolving active ingredients as well as promoting permeation.

### Example 1: Solubility of the active ingredient of colchicine in different types of pressure-sensitive adhesives

Colchicine with different contents was dissolved in pressure-sensitive adhesive systems with different properties. The results are shown in Table 3.

**Table 3 List of solubility of the active ingredient of colchicine in different types of pressure-sensitive adhesives**

| No. | Pressure-sensitive adhesive | Colchicine% | Performance of adhesive solution | Performance of dry colloid matrix | Optical magnifier phenomenon |
|---|---|---|---|---|---|
| 1 | 4098 | 1 | Good solubility, clear | Proper viscosity, transparent | Uniformly dispersed, no crystallization |
| 2 | | 3 | | Proper viscosity, transparent | Uniformly dispersed, no crystallization |
| 3 | | 5 | | Proper viscosity, transparent | Uniformly dispersed, no crystallization |
| 4 | | 7 | | Proper viscosity, semitransparent | Uniformly dispersed, crystallization |
| 5 | | 10 | | Proper viscosity, semitransparent | Uniformly dispersed, crystallization |
| 6 | | 15 | | Proper viscosity, semitransparent | Uniformly dispersed, crystallization |
| 7 | | 20 | | Reduced viscosity, semitransparent | Uniformly dispersed, crystallization |
| 8 | | 30 | | Viscosity loss | Uniformly dispersed, crystallization |
| 9 | | 40 | | Viscosity loss | Uniformly dispersed, crystallization |
| 10 | 2287 | 3 | Good solubility, clear | Proper viscosity, transparent | Uniformly dispersed, no crystallization |
| 11 | | 5 | | Proper viscosity, transparent | Uniformly dispersed, no crystallization |
| 12 | | 7 | | Proper viscosity, semitransparent | Uniformly dispersed, crystallization |
| 13 | 2510 | 3 | Dissolved, clear | Proper viscosity, transparent | Uniformly dispersed, no crystallization |
| 14 | | 5 | | Proper viscosity, transparent | Uniformly dispersed, no crystallization |
| 15 | | 7 | | Proper viscosity, semitransparent | Uniformly dispersed, crystallization |
| 16 | 2051 | 3 | Dissolved, clear | Proper viscosity, transparent | Uniformly dispersed, no crystallization |
| 17 | 2852 | 3 | Good solubility, clear | Proper viscosity, transparent | Uniformly dispersed, no crystallization |
| 18 | | 5 | | Proper viscosity, transparent | Uniformly dispersed, no crystallization |
| 19 | 235A | 5 | Good solubility, clear | Proper viscosity, transparent | Uniformly dispersed, no crystallization |
| 20 | 2516 | 5 | Good solubility, clear | Proper viscosity, transparent | Uniformly dispersed, no crystallization |
| 21 | 2677 | 5 | Good solubility, clear | Proper viscosity, transparent | Uniformly dispersed, no crystallization |
| 22 | 2074 | 5 | Good solubility, clear | Proper viscosity, transparent | Uniformly dispersed, no crystallization |
| 23 | 2196 | 5 | Good solubility, clear | Proper viscosity, transparent | Uniformly dispersed, no crystallization |
| 24 | 900A | 5 | Good solubility, clear | Good solubility, clear | Proper viscosity, transparent |
| 25 | 4302 | 3 | Uniformly dispersed | Proper viscosity, semitransparent | Uniformly dispersed, no crystallization |
| 26 | | 5 | Uniformly dispersed, small particles visible | Proper viscosity, semitransparent | Uniformly dispersed, no crystallization |
| 27 | 6302 | 3 | Good solubility, clear | Good solubility, clear | Proper viscosity, transparent |
| 28 | | 5 | Good solubility, clear | Good solubility, clear | Proper viscosity, transparent |

From the results in Table 3, it can be seen that colchicine and different types of polyacrylic pressure-sensitive adhesives can be miscible and dispersed in the colloid to form a uniform solution. Colchicine exhibits smaller solubility/dispersibility in the silicone pressure-sensitive adhesive 4302, whereas for the polyacrylic acid and silicone composite pressure-sensitive adhesive 6302, colchicine exhibits a solubility similar to that in the polyacrylic pressure-sensitive adhesive alone. The experimental results show that colchicine can satisfy certain solubility and dispersibility in different polyacrylic pressure-sensitive adhesives, silicone pressure-sensitive adhesives or acrylic acid and silicone mixed pressure-sensitive adhesives.

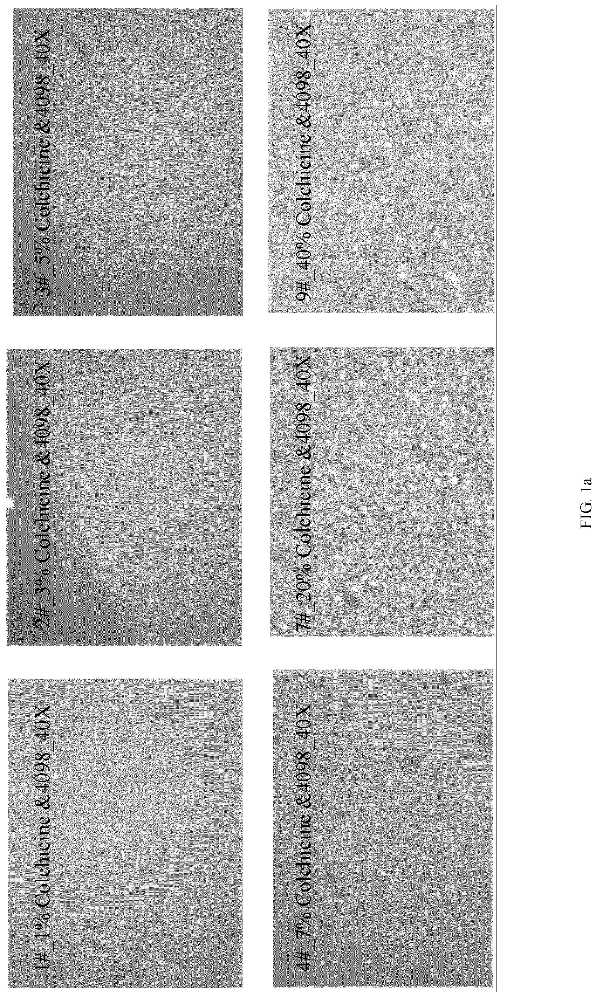

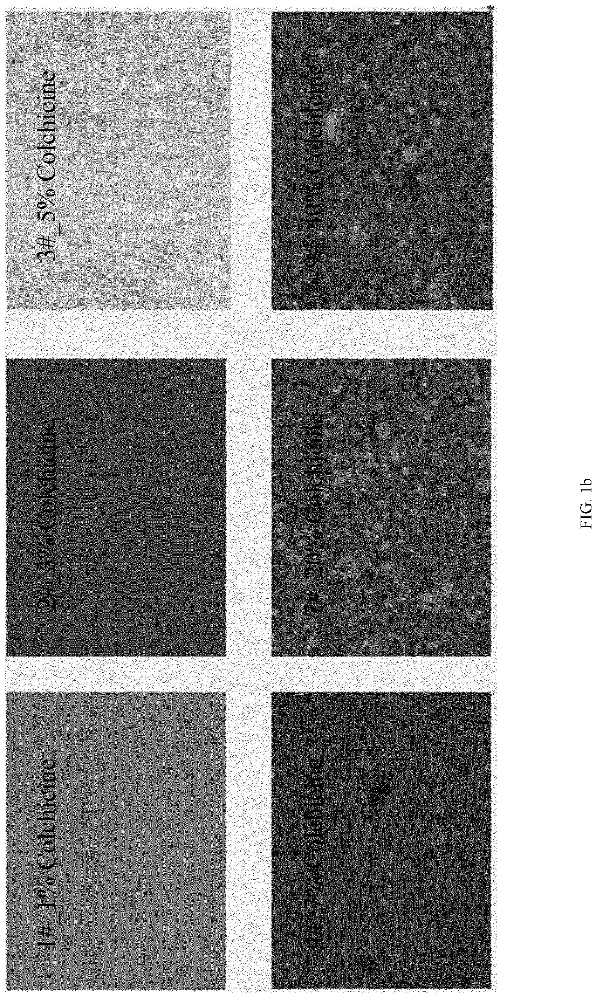

The stability (crystallization or not) of colchicine with different contents in the pressure-sensitive adhesive (the 4098 pressure-sensitive adhesive as an example) for 0 days and 4 months was observed with an optical magnifier. The results are shown in FIG. 1.

FIG. 1a is an observation image for 0 days, from which it can be seen that when the content of colchicine in the 4098 pressure-sensitive adhesive is 5% or less, it is transparent, and when the content is higher, it is semitransparent; and FIG. 1b is an observation image for 4 months later, from which it can be seen that when the content of colchicine in the 4098 pressure-sensitive adhesive is more than 5%, crystallization is precipitated after standing at room temperature for 4 months, and when the content of colchicine is 5% or less, no crystallization is found, and it remains transparent and has good viscosity. The stability results illustrate that the highest solubility of colchicine in typical acrylic pressure-sensitive adhesives can reach about 5% without a permeation enhancer.

### Examples 2-30

The weight percentage contents of each of colchicine, the pressure-sensitive adhesive, and the permeation enhancer in the polymer matrix layer of Examples 2-30 are as shown in Table 4.

**Table 4 List of weight percentage contents of each of colchicine, pressure-sensitive adhesive, and permeation enhancer in polymer matrix layer of Examples 2-30**

| Exam -ple | Pressure-sensitive adhesive | API /% | Menthol /% | Dimethicone /% | Oleic-acid /% | IPP /% | PEG-600 /% | DG-ME /% | HEP /% | TE-OA /% | DPG /% | Oleyl-alcohol /% | DMI /% | Permeation enhancing effect |
|---|---|---|---|---|---|---|---|---|---|---|---|---|---|---|
| 2 | 4098 | 3.00 | | | | | | | | | 2.00 | | | B |
| 3 | 2074 | 3.00 | | | | | | | | | 2.00 | | | B |
| 4 | 2852 | 3.00 | | | | | | | | | 2.00 | | | B |
| 5 | 4302 | 3.00 | | | | | | | | | 2.00 | | | B |
| 6 | 4302 | 2.00 | 2.00 | | | | | | | | | | | A |
| 7 | 4302 | 2.00 | | 2.00 | | | | | | | | | | A |
| 8 | 4302 | 2.00 | | | 2.00 | | | | | | | | | A |
| 9 | 4302 | 2.00 | | | | 2.00 | | | | | | | | A |
| 10 | 4302 | 2.00 | | | | | 2.00 | | | | | | | A |
| 11 | 4302 | 2.00 | | | | | | 2.00 | | | | | | c |
| 12 | 4302 | 2.00 | | | | | | | 2.00 | | | | | c |
| 13 | 4302 | 2.00 | | | | | | | | 2.00 | | | | c |
| 14 | 4302 | 2.00 | | | | | | | | | 2.00 | | | c |
| 15 | 4302 | 2.00 | | | | | | | | | | 2.00 | | B |
| 16 | 4302 | 0.50 | | | | | | 2.00 | | | | | 2.00 | B |
| 17 | 4302 | 3.00 | | | | | | 2.00 | | | | | | c |
| 18 | 4302 | 1.00 | | | | | | | | 0.65 | | | | C |
| 19 | 4302 | 2.00 | | | | | | 8.00 | | | | | | c |
| 20 | 6302 | 2.00 | 2.00 | | | | | | | | | | | A |
| 21 | 6302 | 2.00 | | | 2.00 | | | | | | | | | A |
| 22 | 6302 | 2.00 | | | | | | 2.00 | | | | | | A |
| 23 | 6302 | 2.00 | | | | | | | | 0.05 | | | | C |
| 24 | 6302 | 2.00 | | | | | | | | 0.25 | | | | C |
| 25 | 6302 | 2.00 | | | | | | | | 0.65 | | | | C |
| 26 | 6302 | 2.00 | | | | | | | | | 0.50 | | | C |
| 27 | 6302 | 2.00 | | | | | | | | | | 2.00 | | A |
| 28 | 6302 | 1.00 | | | | | | | | 0.25 | | | | C |
| 29 | 6302 | 0.50 | | | | | | | | 0.25 | | | | C |
| 30 | 900A & 4302 (1:1, w/w) | 2.00 | | | | | | 4.00 | | | | | | C |
| A = permeation substantially unchanged; B = permeation slightly increased; C = permeation obviously increased | | | | | | | | | | | | | | |

It can be seen from the results in Table 4 that adding different types of permeation enhancers has different effects on the change of permeation efficiency. For non-alcohol permeation enhancers such as menthol, dimethicone, oleic acid, IPP, PEG600, DMI, etc., there is no obvious permeation enhancing effect in either acrylic pressure-sensitive adhesives or silicone pressure-sensitive adhesives; and for alcohols or alcohol derivatives such as DGME, HEP, TEOA, and DPG, there is obvious permeation enhancing effect. However, the permeation enhancing effect is related to the structure of alcohols, such as oleyl alcohol and PEG600, which include alcohol hydroxyl groups, but do not show permeation enhancing effect or have a slight permeation enhancing as the increase of the chain length.

### Examples 31-44

Each component and weight percentage content of the patch containing colchicine in Examples 31-44 are as shown in Table 5.

**Table 5 Each component and weight percentage content of patch containing colchicine in Examples 31-44**

| Example | Pressure-sensitive adhesive | API/% | TEOA/% | Backing film | Permeation ability | Notes |
|---|---|---|---|---|---|---|
| 31 | 4302 | 2.00 | 0.65 | 3M Scotchpak^{™} 9738 | A | |
| 32 | 4302 | 2.00 | 0.65 | 3M Scotchpak^{™} 9754 | c | |
| 33 | 4302 | 2.00 | 0.65 | 3M^{™} CoTran^{™} 9720 | c | |
| 34 | 4302 | 2.00 | 0.65 | 3M Scotchpak^{™} 9733 | c | |
| 35 | 4302 | 2.00 | 0.65 | 3M^{™} Scotchpak^{™} 9723 | B | |
| 36 | 4302 | 2.00 | 0.65 | Elastic fabric 6015A from Xiaoshan | c | Peeling failure |
| 37 | 4302 | 2.00 | 0.65 | Woven fabric EW2083 from Vilene in Japan | C | Peeling failure |
| 38 | 4302 | 2.00 | 0.65 | 3601 from Shanghai Yingke | B | |
| 39 | 4302 | 2.00 | 0.65 | 3M CoTran^{™} 9701 | c | |
| 40 | 6302 | 2.00 | 0.65 | 3M Scotchpak^{™} 9738 | A | |
| 41 | 6302 | 2.00 | 0.65 | Woven fabric EW2083 from Vilene in Japan | B | |
| 42 | 6302 | 2.00 | 0.65 | 3601 from Shanghai Yingke | B | |
| 43 | 6302 | 2.00 | 0.65 | 3M CoTran^{™} 9701 | B | |
| 44 | 6302 | 2.00 | 0.65 | 3M Scotchpak^{™} 9733 | c | |
| Description | Permeation ability: To investigate the pasting on Bama miniature pigs for 12 hr, A = normal permeation (fluctuation of 10%); B = permeation decreased (decreased by about 50%); C = permeation decreased severely (decreased to below 20%) | | | | | |

From the results in Table 5, it can be seen that backing films with different properties will greatly affect the permeation performance and pasting performance. 3M Scotchpak^{™} 9754 (polyester PET), 3M^{™} CoTran^{™} 9720 & 3601 from Shanghai Yingke (polyethylene PE), 3M Scotchpak^{™} 9733 (polyester PET and polyvinyl acetate EVA mixed film), the elastic fabric 6015A from Xiaoshan, non-woven fabric EW2083 from Vilene in Japan and 3M CoTran^{™} 9701 (polyurethane, PU) leads to a great decrease in the permeation ability, indicating that the non-closed backing films will affect the permeation severely. This is possibly because that the moisture from skin's respiration at the pasting site passes through the colloidal matrix layer, and then through the backing layer. When the water passes through the colloidal matrix layer, the solubility of the active ingredient in the colloidal matrix increases due to the excellent water solubility of colchicine, which leads to a decrease in its thermodynamic diffusion ability, and thus showing a significant decrease in the permeation ability. For completely unclosed backings such as elastic fabrics or non-woven fabrics, peeling failure is further caused. Whereas 3M Scotchpak^{™} 9738, a closed backing film including aluminum, has a good permeation performance, which is possibly because the formation of a closed system, so that the moisture can not completely pass through the colloidal matrix layer, thus having little influence on the active ingredient, and showing no obvious change in the permeation ability.

### Comparative Examples 1-12

The weight percentage contents of each of colchicine, the pressure-sensitive adhesive, and the permeation enhancer in the polymer matrix layer of Comparative Examples 1-12 are as shown in Table 6.

**Table 6 List of weight percentage contents of each of colchicine, pressure-sensitive adhesive, and permeation enhancer in polymer matrix layer of Comparative Examples 1-12**

| Comparative Example | Pressure-sensitive adhesive | API/% | Ethylene glycol/% | Diethylene glycol/% | Propylene glycol/% | Glycerol/% | 1,3-butanediol/% | Colloid performance |
|---|---|---|---|---|---|---|---|---|
| 1 | 4302 | 2 | 1 | | | | | Destroyed |
| 2 | 4302 | 2 | 2 | | | | | Destroyed |
| 3 | 4302 | 2 | | 2 | | | | Destroyed |
| 4 | 4302 | 2 | | | 2 | | | Destroyed |
| 5 | 4302 | 2 | | | | 2 | | Destroyed |
| 6 | 4302 | 2 | | | | 4 | | Destroyed |
| 7 | 4302 | 2 | | | | 6 | | Destroyed |
| 8 | 6302 | 2 | 2 | | | | | Destroyed |
| 9 | 6302 | 2 | | 2 | | | | Destroyed |
| 10 | 6302 | 2 | | | 2 | | | Destroyed |
| 11 | 6302 | 2 | | | | 2 | | Destroyed |
| 12 | 6302 | 2 | | | | | 2 | Destroyed |

From the results in Table 6, it can be seen that the addition of short-branched or straight chain alcohol permeation enhancers will destroy the colloidal performance. That is because the short-branched or straight chain alcohol permeation enhancers have a higher polarity, while the silicone pressure-sensitive adhesives have an extremely low polarity. Although colchicine can be uniformly dissolved or dispersed in the pressure-sensitive adhesive system due to the role of the permeation enhancers, part of the adhesive solution is changed from a uniform solution to a clumpy, insoluble jelly-like mixture because of the compatibility issues between the permeation enhancer and the pressure-sensitive adhesives, thus preventing the completion of the coating.

### Preparation method of Example 2:

The preparation method for the patch containing colchicine is as follows:
Step 1): firstly, weighing a prescribed amount of colchicine and a permeation enhancer, adding them into anhydrous ethanol with the same weight and stirring them to dissolve, with the stirring time depending on the sample amount, generally 15-30 minutes, to obtain a mixed solution;
Step 2): weighing a prescribed amount of polymer pressure-sensitive adhesive, dropping the mixed solution of colchicine and the permeation enhancer in ethanol obtained in Step 1) slowly into the pressure-sensitive adhesive while keeping stirring, with the dropping time depending on the amount of the solution of colchicine in ethanol, generally 5-30 minutes, and then continuing stirring for 10-30 minutes after dropping to uniformly disperse all the adhesive matrix to obtain an adhesive solution;
Step 3): uniformly stirring the adhesive solution obtained in Step 2), and then coating the stirred adhesive solution on the 3M Scotchpak^{™} 9744 release film with the coating thickness depending on the final clinical use, and drying the coated polymer matrix at 35-50°C for 5-15 minutes through an oven with an air exhaust function to remove the organic solvent, with the specific drying temperature and time depending on the coating speed and solvent residue; and
Step 4): compositing a product obtained in Step 3) with the 3M Scotchpak^{™} 9738 backing film to obtain a composite product, and finally, die-cutting the composite product according to use requirements to obtain composite products with required specifications, and packaging the composite products with required specifications to obtain patches containing colchicine.

The preparation methods of Examples 3 to 44 are the same as those of Example 2.

The preparation methods of Comparative Examples 1-12 are the same as those of Example 2.

### Test Example 1: Solubility test of colchicine

About 2 g of the colchicine raw material was weighed and placed in a 100 ml brown volumetric flask, into which 10 ml of a solvent was added. The mixture was uniformly mixed, placed in a thermostatic oscillator with a water bath at 25°C, and observed periodically, into which the raw material was continuously added once the solution became clear. After being oscillated in the thermostatic oscillator with a water bath at 25°C for 24 h, the solution still had precipitates, indicating that the solution was saturated. After the saturated solution was centrifuged in a centrifuge, the supernatant was taken and diluted to a suitable concentration, the concentration of which was then determined using the external standard method, and the solubility was calculated. The results are shown in Table 7.

**Table 7 List of solubility of colchicine in different solvents**

| No. | Solvent | Solubility |
|---|---|---|
| 1 | water | > 1.00 |
| 2 | ethanol | 0.59 |
| 3 | Ethyl acetate | 0.00048 |
| 4 | Ethanol:ethyl acetate (1:1, v/v) | 0.38 |
| 5 | 1M solution of TEOA (triethanolamine) in ethanol | 0.74 |
| 6 | DGME (diethylene glycol monoethyl ether) | 0.35 |

From the results in Table 7, it can be seen that colchicine has the greatest solubility in water, and then has a certain solubility in alcohol, alcohol ether or alcohol amine solvent, and little solubility in ethyl acetate.

### Test Example 2: Long-term stability test

The patch of Example 24 was tested in a long-term stability environment. The test was carried out in an environment with a temperature of 30°C and a humidity of 60%±5% RH. The results are shown in Table 8.

**Table 8 List of test results of patch of Example 24 in long-term stability environment**

| Related substance (%) | Limit requirement (%) | Time (month) | | | | | |
|---|---|---|---|---|---|---|---|
| | | 0 | 1 | 3 | 6 | 9 | 12 |
| c | 0.15 | Not detected | Not detected | Not detected | Not detected | Not detected | Not detected |
| G | 0.15 | Not detected | Not detected | Not detected | Not detected | Not detected | Not detected |
| Maximum unknown impurity | 0.50 | 0.08 | 0.06 | 0.10 | 0.13 | 0.14 | 0.09 |
| Unknown total impurity | 5.00 | 0.08 | 0.11 | 0.14 | 0.15 | 0.16 | 0.15 |

From the results in Table 8, it can be seen that photodegradation products C and G and other unknown impurities were not produced during the placement of the sample of the patch containing colchicine for stability test, indicating that the transdermal drug delivery system of the present disclosure is stable.

### Test Example 3: In vitro permeation test

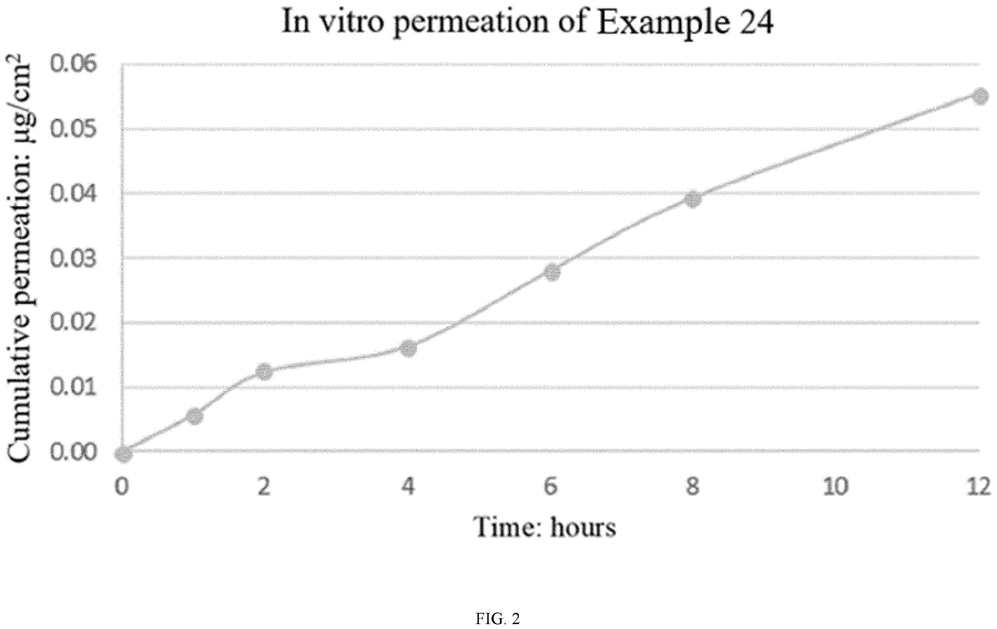

The *in vitro* permeation test of the patch of Example 24 was determined by a Franz vertical diffusion cell. The back skin of a 30-day-old Bama miniature pig was used, with a thickness of 0.3 mm. The receiving solution was PBS with a pH of 7.4, the volume of the receiving cell was 7 mL, the set temperature was 32 ± 0.1°C, and the stirring rate was 200 revolutions/minute. Samples were taken at 1 h, 2 h, 4 h, 6 h, 8 h and 12 h, respectively, with 3 mL each time, and then the isothermal blank receiving solution was added. Each group of samples was tested in triplicate. Based on the results, the cumulative permeation amount at each time point was calculated, as shown in FIG. 2.

From the results in FIG. 2, it can be seen that the moisture has a significant influence on the permeation behavior of the patch containing colchicine. Due to the high water solubility of colchicine, the moisture in the receiving medium permeates into the colloidal matrix of the patch, which leads to a significant decrease in the permeation ability (see Test Example 4, the *in vivo* back permeation data of the Bama miniature pig).

### Test Example 4: In vivo permeation test in Bama miniature pigs

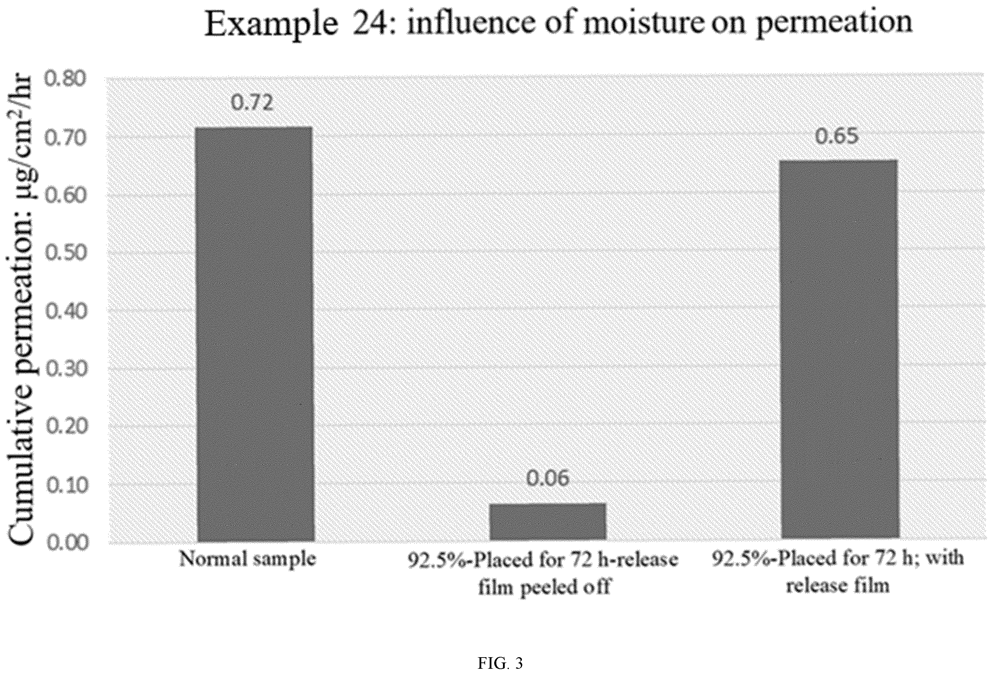

The *in vivo* permeation test of the patch of Example 24 investigated the influence of the moisture on permeation. Test scheme: The samples were placed in a high humidity environment with 92.5% RH for 72 hr in advance, where one had a release film, and the other had a release film peeled off in advance to expose the colloidal matrix directly to the high humidity environment. A 30-day-old Bama miniature pig was subjected to an *in vivo* back percutaneous test. The results are shown in FIG. 3.

From the results in FIG. 3, it can be seen that the moisture has a significant influence on the permeation behavior of the patch containing colchicine. Because of the high water solubility of colchicine, the water in the environment has a significant influence on the permeation behavior, and the water absorbed by the patch from the environment will inhibit the permeation behavior of colchicine; after the samples were treated in the high humidity environment, the *in vivo* permeation of the samples with a release film on the back of the Bama miniature pig decreases slightly, and the *in vivo* permeation of the samples without a release film decreases greatly.

### Test Example 5: Local concentration test of joint capsule in rats

Seventy-two male SD rats were randomly divided into six groups according to their body weight, which were used as test group and a model control group. The test group was divided into low, medium and high dose groups of Test prescription 1, a group of Test prescription 2 and a colchicine intragastric control group. The test group and the model control group had 12 rats in each group, and each group was randomly divided into two subgroups, i.e., subgroups 1 and 2, respectively, with 6 rats in each subgroup. Another 6 unmodeled rats were taken as a normal control group. The specific grouping is shown in Table 9, where the low, medium and high dose groups of Test prescription 1 correspond to Examples 29, 28 and 24, respectively. The group of Test prescription 2 corresponds to Example 18.

**Table 9 Grouping list of rats**

| Group No. | Group type | Number of animals | Test object | Dose |
|---|---|---|---|---|
| 1 | Normal control group | 6 | Blank patch | NA |
| 2 | Model control group | 6+6 | Blank patch | NA |
| 3 | Low dose group of Test prescription 1 | 6+6 | Test prescription 1 | 0.5×1.5 cm (0.5%) |
| 4 | Medium dose group of Test prescription 1 | 6+6 | Test prescription 1 | 0.5×1.5 cm (1%) |
| 5 | High dose group of Test prescription 1 | 6+6 | Test prescription 1 | 0.5×1.5 cm (2%) |
| 6 | Group of Test prescription 2 | 6+6 | Test prescription 2 | 0.5×1.5 cm (1%) |
| 7 | Colchicine gavage control group | 6+6 | Colchicine tablet | 0.25 mg/kg |

The low, medium and high dose groups of Test prescription 1 and the group of Test prescription 2 were administrated at 4 and 28 h after modeling, respectively, and were pasted at their ankle joints with the corresponding test samples (about 0.75 cm², 0.5×1.5 cm, while being administrated by pasting, the rats were restrained with gauzes and adhesive tapes on their trunks, and kept in single cages to prevent them from biting the patch by themselves or each other. If the patch dropped off during the test, it should be recorded and supplemented). After being administrated by pasting for 12 h, the rats were freed from administration and trunk restraint. The normal control group and the model control group were pasted with blank patches at the same time and in the same way.

The colchicine gavage control group was administrated once at 4, 16, 28 and 40 h after modeling, respectively, with 0.25 mg/kg each time and the administration volume of 5 mL/kg.

Subgroup 1 and Subgroup 2 were dissected at 19 h and 43 h after modeling, respectively. The normal control group was dissected at 43 h after modeling. The colchicine concentration in the joint capsule tissue was detected after homogenization. The results are as shown in Table 10.

**Table 10 List of concentration of colchicine in joint capsule tissues of rats in each of test groups and control group**

| Concentration of colchicine in joint capsule tissue | | | | |
|---|---|---|---|---|
| Group | Concentration (ng/mL) | | | |
| | 19H | | 43H | |
| | Average | SD | Average | SD |
| Low dose group of Test prescription 1 | 86.6 | 57.9 | 225.9 | 181.6 |
| Medium dose group of Test prescription 1 | 103.3 | 46.6 | 147.0 | 89.7 |
| High dose group of Test prescription 1 | 110.1 | 72.8 | 392.0 | 174.5 |
| Group of Test prescription 2 | 70.5 | 89.9 | 101.2 | 60.9 |
| Colchicine gavage control group | 11.3 | 7.9 | 14.1 | 13.7 |

From the results in Table 10, it can be seen that the colchicine concentration in the local joint capsule of rats after patch pasting is much higher than that after gavage.

### Test Example 6: Irritant test in rabbits

Four Japanese white rabbits were used as a blank control group, a low dose group of Test prescription 1 (Example 29), a middle dose group of Test prescription 1 (Example 28) and a high dose group of Test prescription 1 (Example 24), respectively. Each of the white rabbits were administrated on four parts of the back. One or two days before administration, the hair on the administration site was removed by using a depilatory device, with a square depilatory area of about 5*5 cm². The depilatory cream was applied to the depilatory site, so that it could be evenly applied to the depilatory site to depilate for approximately 40-60 s. Then, the depilatory site was rinsed with warm water to ensure complete removal of the depilatory cream, and had its water absorbed with a gauze.

Before administration, the skin at the depilatory site was photographed and preserved, and the test sample with a diameter of 3 cm prepared in advance was pasted on the administration site, and removed after being pasted for 8 hours. The skin reaction was observed with naked eyes at 60 minutes, 24 hours, 48 hours and 72 hours after the test sample was removed, and the irritation reaction and edema degree were scored according to Table 11.

**Table 11 Scoring criteria of skin irritation reaction**

| Irritation reaction | Score |
|---|---|
| **Erythema** | |
| No erythema | 0 |
| Mild erythema (barely visible) | 1 |
| Moderate erythema (obviously visible) | 2 |
| Severe erythema | 3 |
| Purple erythema to mild eschar formation | 4 |

| **Edema** | |
|---|---|
| No edema | 0 |
| Mild edema (barely visible) | 1 |
| Moderate edema (obvious bulge) | 2 |
| Severe edema (skin bulge of 1 mm with clear outline) | 3 |
| Serious edema (skin bulge of more than 1 mm and expansion) | 4 |
| Maximum total score | 8 |

The average of the integrals of the skin reaction at each time point for each group was calculated, and an evaluation of the irritation intensity was conducted according to Table 12.

**Table 12 Evaluating criteria of skin irritation intensity**

| Score | Evaluation |
|---|---|
| 0~0.49 | No irritant |
| 0.5~2.99 | Mild irritant |
| 3.0~5.99 | Moderate irritant |
| 6.0~8.00 | Severe irritant |

After being administrated, the white rabbits in each group were given irritant scores, in which no erythema and edema were observed in the blank control group, the low dose group of Test prescription 1 (Example 29), the middle dose group of Test prescription 1 (Example 28) and the high dose group of Test prescription 1 (Example 24) at each time point after administration, and the skin irritation intensity evaluation were all 0 score. So, it can be determined that the patch containing colchicine has no irritant when the weight percentage content of colchicine is 0.5%, 1% and 2%.

### Test Example 7: Study on distribution of colchicine in local tissue of Bama miniature pigs

Eight Bama miniature pigs of the same sex (all male) were randomly divided into two groups according to their body weight, with 4 pigs/group. Group 1, as a patch group, was administrated the patch containing colchicine of the present disclosure at the joints of limbs by transdermally pasting in 4 patches/pig in a single time, where the patch was prepared by Example 30, with a patch specification of 5 cm*7 cm/patch. Group 2, as a tablet group, was administrated the colchicine tablets (purchased from Takada Pharmaceutical Co., Ltd., Batch No. UX10) by oral gavage administration in 2 tablets/pig (1 mg/pig) in a single time.

**Table 13 Administration information in each group**

| **Group No.** | **Group type** | **Animal number** | **Route of administration** | **Preparation specification** | **Administrating amount** |
|---|---|---|---|---|---|
| 1 | Colchicine patch | 1101-1104 | transdermally administrating at joints of limbs | 0.085 mg/cm² | 140 cm²/pig (35 cm²/limb) |
| 2 | Colchicine tablet | 1205-1208 | PO | 0.5 mg/tablet | 2 tablets/pig |

| | | | | | |
|---|---|---|---|---|---|
| Note: The first digit of the animal number represents gender (1 is for male, and 2 is for female); the second digit represents the group type (1 is for the first group, 2 is for the second group, and so on); the last two digits represent the animal serial number. | | | | | |

One animal in each group was dissected at 0.5 h, 2 h, 4 h and 8 h after administration, and its plasma, liver, kidney, stomach, duodenum, rectum, joint capsule of limbs (left anterior, right anterior, left posterior and right posterior), skin at joints and subcutaneous tissue were collected. The tissue samples collected from the Bama miniature pigs were weighed, into which then acetonitrile was added in a ratio of 1:3 (w:v, g/mL), and the mixture was homogenized on a high-throughput tissue grinder to prepare a Bama miniature pig tissue homogenate.

The concentration of colchicine in each tissue sample of the Bama miniature pigs was quantitatively detected by the LC-MS/MS method.

The distribution of colchicine in each of the collected tissues at different time points in the Bama miniature pigs was statistically analyzed, and pharmacokinetic parameters in each of the tissues and plasma were calculated.

### Results:

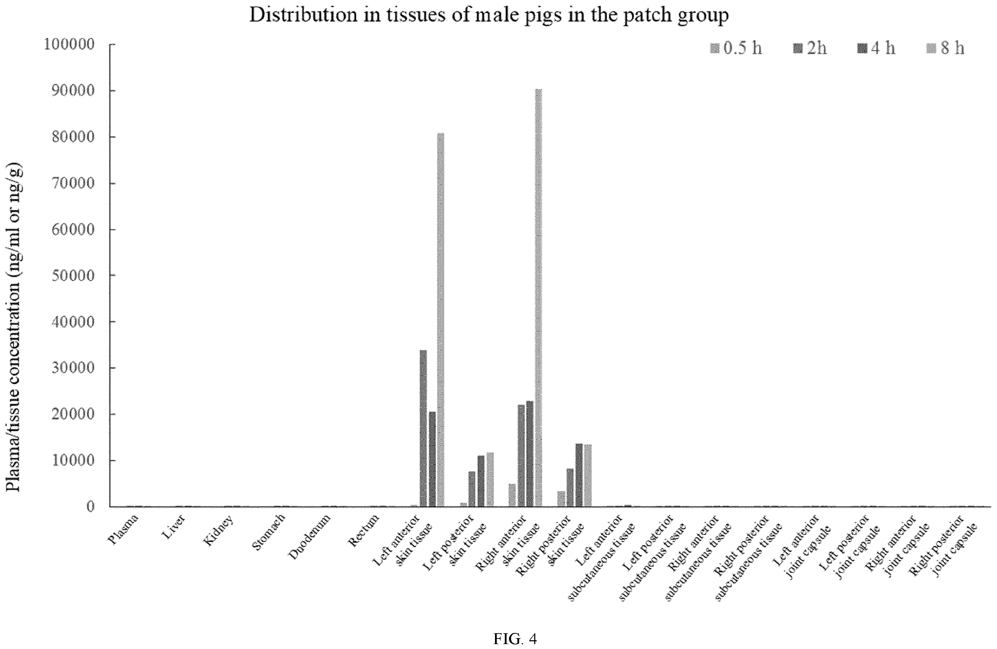

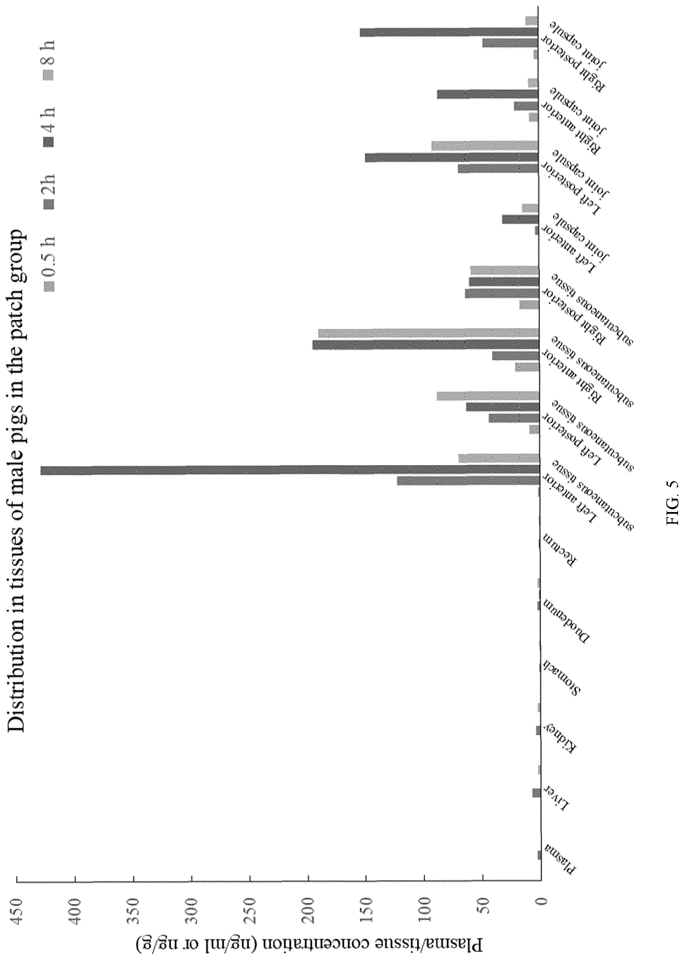

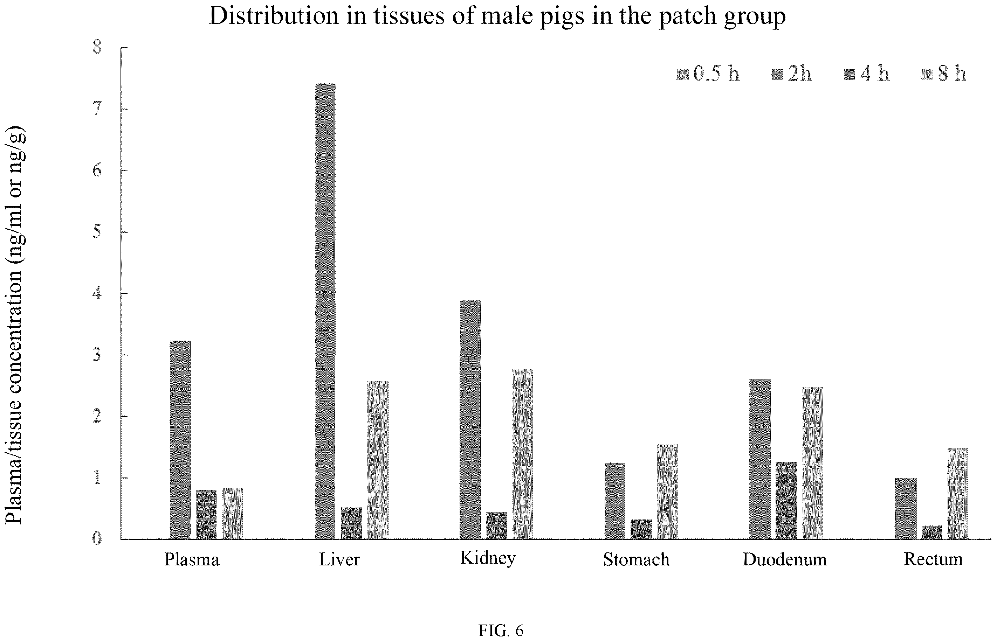

For Group 1 as the patch group, after the patches containing colchicine of the present disclosure were transdermally administrated to the joints of limbs of the Bama miniature pigs, as shown in FIGs. 4-6, colchicine was mainly distributed in the skin tissue, followed by the subcutaneous tissue and joint capsule, and relatively less in the plasma, liver, kidney and gastrointestinal tract; the concentrations of colchicine in each tissue at different time points were ranked as follows: at 0.5 h, the concentration in the limb skin tissue > limb subcutaneous tissue > limb joint capsule, and the concentrations in the plasma, liver, kidney, stomach, duodenum and rectum were all lower than the quantitative lower limit; at 2 h, the concentration in the limb skin tissue > limb subcutaneous tissue > limb joint capsule > liver > kidney > plasma > duodenum > stomach > rectum; at 4 h, the concentration in the limb skin tissue > limb subcutaneous tissue > limb joint capsule > duodenum > plasma > liver > kidney > stomach > rectum; and at 8 h, the concentration in the limb skin tissue > limb subcutaneous tissue > limb joint capsule > kidney > liver > duodenum > stomach > rectum > plasma. The total exposures (AUC_{0-8 h}) were ranked as follows: AUC_{0-8 h} in the limb skin tissue > limb subcutaneous tissue > limb joint capsule > liver > duodenum > kidney > plasma > stomach > rectum.

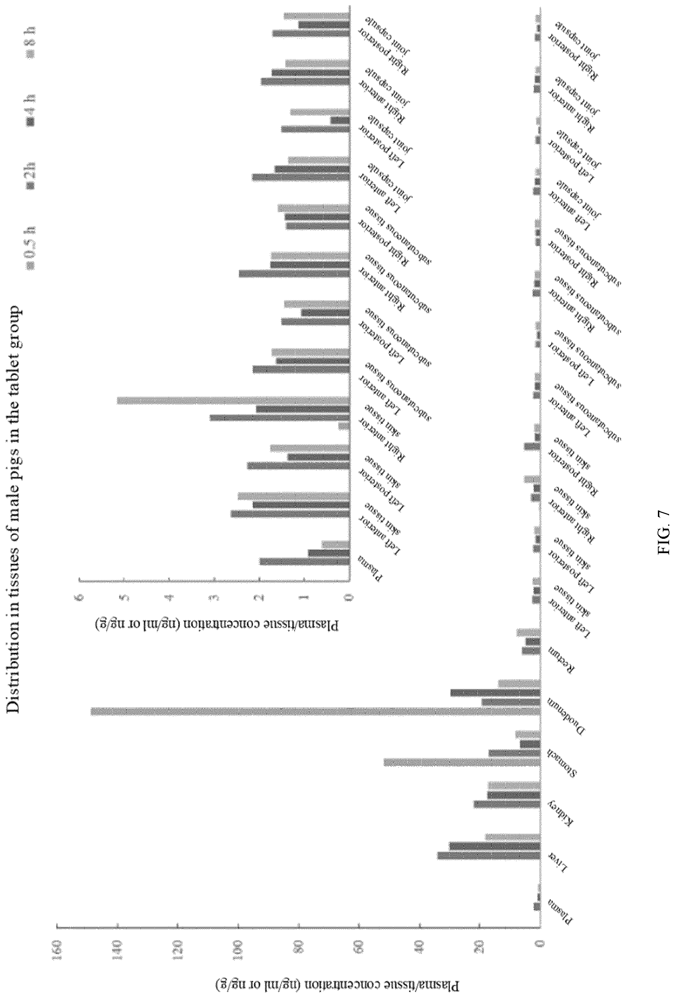

For Group 2 as the tablet group, after the colchicine tablets were administrated by oral gavage to the Bama miniature pigs, as shown in FIG. 7, colchicine was mainly distributed in the liver, kidney and gastrointestinal tract, and relatively less in the plasma, skin tissue, subcutaneous tissue and joint capsule; at 0.5 h, the concentration in the duodenum > stomach > limb skin tissue, and the concentrations in the plasma, liver, kidney, rectum, limb joint capsule and limb subcutaneous tissue were all lower than the quantitative lower limit; at 2 h, the concentration in the liver > kidney > duodenum > stomach > rectum > limb skin tissue > plasma > limb subcutaneous tissue > limb joint capsule; at 4 h, the concentration in the liver > duodenum > kidney > stomach > rectum > limb skin tissue > limb subcutaneous tissue > limb joint capsule > plasma; at 8 h, the concentration in the liver > kidney > duodenum > stomach > rectum > limb skin tissue > limb subcutaneous tissue > limb joint capsule > plasma. The total exposures (AUC_{0-8 h}) were ranked as follows: AUC_{0-8 h} in the duodenum > liver > kidney > stomach > rectum > limb skin tissue > limb subcutaneous tissue > limb joint capsule > plasma.

Comparing the patch group of Bama miniature pigs with the tablet group, in the liver, kidney, duodenum and rectum, the Cₘₐₓ ratios were within a range of 0.02-0.22, and the AUC_{0-8 h} ratios were within a range of 0.05-0.13, which were all <1; while in the limb skin tissue, limb subcutaneous tissue and limb joint capsule, the Cₘₐₓ ratios were within a range of 57.38-12866.49, and the AUC_{0-8 h} ratios were within a range of 41.54-11019.52.

### Conclusion:

After the patches containing colchicine (11.9 mg/pig) were transdermally administrated to the Bama miniature pigs, colchicine were mainly distributed in the skin tissue, followed by the subcutaneous tissue and joint capsule, and relatively less in the plasma, liver, kidney and gastrointestinal tract; after the colchicine tablets (1 mg/pig) were administrated by oral gavage to the Bama miniature pigs, colchicine was mainly distributed in the liver, kidney and gastrointestinal tract, and relatively less in the plasma, limb skin tissue, limb subcutaneous tissue and limb joint capsule; in the patch group, the Cₘₐₓ and AUC_{0-8 h} of the liver, kidney, duodenum and rectum were all lower than those in the tablet group, whereas the Cₘₐₓ and AUC_{0-8 h} of the limb skin tissue, limb subcutaneous tissue and limb joint capsule were much higher than those in the tablet group.

It should be understood that the above embodiments are all exemplary, and are not intended to cover all possible implementations included in the claims. Various modifications and changes can be made on the basis of the above embodiments without departing from the scope of the present disclosure. Besides, various technical features of the above embodiments can be arbitrarily combined to form other embodiments of the present disclosure that may not be explicitly described. Therefore, the above-mentioned embodiments only describe several implementations of the present disclosure, and do not limit the protection scope of the patent of the present disclosure.

### Industrial practicability

The present disclosure provides a patch containing colchicine, a preparation method and a use thereof, where the patch includes a polymer matrix layer. The polymer matrix layer includes the following components in weight percentage: 0.1%-5.0% of colchicine, 0.005%-15% of a permeation enhancer, and 80%-99% of a pressure-sensitive adhesive. The patch containing colchicine of the present disclosure can achieve transdermal permeation of an amount sufficient for achieving treatment effect, features no local irritant during pasting and after pasting, no gastrointestinal side effects, and high patient compliance, and has good economic value and application prospect.

## Claims

1. A patch containing colchicine, wherein the patch comprises a polymer matrix layer comprising the following components in weight percentage:
| | |
|---|---|
| colchicine | 0.1%-5.0%, |
| a permeation enhancer | 0.005%-15%, and |
| a pressure-sensitive adhesive | 80%-99%; |
preferably, in the polymer matrix layer, a weight percentage of the colchicine is 0.25%-4.0%, further preferably 0.50%-3.5%, and more preferably 0.50%-3.0%;
preferably, in the polymer matrix layer, a weight percentage of the permeation enhancer is 0.01%-12%; and/or
preferably, in the polymer matrix layer, a weight percentage of the pressure-sensitive adhesive is 85%-98%.

2. The patch containing colchicine according to claim 1, wherein the patch further comprises a backing layer;
preferably, the patch further comprises a protective layer, and the polymer matrix layer is located between the backing layer and the protective layer.

3. The patch containing colchicine according to claim 1 or 2, wherein the permeation enhancer is selected from reagents capable of dissolving colchicine and having a boiling point greater than or equal to 150°C;
preferably, the permeation enhancer is selected from one or more of alcohol, alcohol amine and alcohol ether;
preferably, the permeation enhancer has a boiling point greater than or equal to 180°C;
preferably, the alcohol is selected from monohydric alcohol and/or dihydric alcohol;
preferably, the monohydric alcohol is selected from one or more of lauryl alcohol, oleyl alcohol and terpineol;
preferably, the dihydric alcohol is selected from hexanediol;
preferably, the alcohol amine is selected from one or more of tromethamine, ethanolamine, diethanolamine, triethanolamine, N-hydroxyethyl piperidine, N-hydroxyethyl pyrrolidine, N-hydroxyethyl piperazine, N,N-dibutylaminoethanol and N,N-diethylaminoethanol; and
preferably, the alcohol ether is selected from one or more of diethylene glycol monoethyl ether, diethylene glycol monobutyl ether, diethylene glycol monohexyl ether, dipropylene glycol methyl ether, dipropylene glycol butyl ether and dipropylene glycol.

4. The patch containing colchicine according to claim 2, wherein the backing layer is selected from a closed backing film or a non-closed backing film;
preferably, the backing layer is selected from the closed backing film;
preferably, the closed backing film is selected from a light-proof closed backing film;
preferably, the closed backing film is selected from a polyester-polyethylene composite film lined with metal aluminum;
preferably, the closed backing film has a thickness of 20-100 µm; and
preferably, the protective layer is selected from a release film.

5. The patch containing colchicine according to claim 1 or 2, wherein the pressure-sensitive adhesive is selected from one or more of an acrylic pressure-sensitive adhesive or a silicone pressure-sensitive adhesive; and more preferably, the pressure-sensitive adhesive is selected from one or two of an acrylic pressure-sensitive adhesive or a silicone pressure-sensitive adhesive.

6. The patch containing colchicine according to claim 1 or 2, wherein the polymer matrix layer comprises colchicine with a content ranging from 10 µg/cm² to 150 µg/cm²; and
preferably, an administration area of the patch containing colchicine is 5 cm²-100 cm².

7. A method for preparing a patch containing colchicine according to any one of claims 1 to 6, comprising the following Steps:
(1) dissolving a prescribed amount of colchicine and a permeation enhancer in an organic solvent to obtain a mixed solution;
(2) dropping the mixed solution obtained in Step (1) into a prescribed amount of a pressure-sensitive adhesive to obtain an adhesive solution;
(3) coating the adhesive solution obtained in Step (2) on a protective layer, and removing the organic solvent; and
(4) compositing a product obtained in Step (3) with a backing layer, and die-cutting it to obtain the patch containing colchicine.

8. A method for preparing a patch containing colchicine according to any one of claims 1 to 6, comprising the following Steps:
(1') adding and mixing a prescribed amount of colchicine, a permeation enhancer and a pressure-sensitive adhesive into an organic solvent to obtain an adhesive solution;
(2') coating the adhesive solution obtained in Step (1') on a protective layer, and removing the organic solvent; and
(3') compositing a product obtained in Step (2') with a backing layer, and die-cutting it to obtain the patch containing colchicine.

9. The method according to claim 7 or 8, wherein:
the organic solvent in the Step (1) and/or the Step (1') is selected from one or more of methanol, ethanol or isopropanol; and
preferably, a specific operation of removing the organic solvent in the Step (3) and/or the Step (2') is: removing the organic solvent by drying at 35-50°C.

10. A use of a patch containing colchicine according to any one of claims 1 to 6 in preparing a medicament for preventing and/or treating gout and/or pericarditis.
